# EUROPEAN PATENT APPLICATION

(11) **EP 1 442 694 A1**
(43) Date of publication of application: **04.08.2004**
(21) Application number: 04001934.1
(22) Date of filing: 29.01.2004
(51) Int. Cl.: A61B 1/005

(54) **Composite flexible endoscope insertion shaft with tubular substructure**

(30) Priority: 29.01.2003 US 443491 P; 27.01.2004 US 766295
(71) Applicant: Karl Storz Endovision, Inc., Charlton, MA 01507 (US)
(72) Inventor: Horne, Guy E. Jr., Dudley, MA 01571 (US); Barry, James P., Charlton, MA 01507 (US)
(74) Representative: Heuckeroth, Volker, Dr.

(57) **Abstract**

An endoscope insertion shaft is disclosed comprising a tubular member (102) having an axis and including at least one aperture (118) for imparting desirable mechanical characteristics to the shaft. A composite, laminated or fused material comprises at least one layer encasing or jacketing the tubular member.

## Description

### Related Applications

This application claims the benefit of provisional U.S. Patent application S.N. 60/443,491, filed on January 29, 2003, which is incorporated herein by reference thereto as if set forth at length.

### Field Of The Invention

The invention relates to a composite flexible endoscope insertion shaft with tubular substructure. The endoscope insertion shaft made according to this invention comprises a composite material, the innermost layer being a tubular member, which provides for improved flexibility.

### Background Of The Invention

Conventional endoscope insertion shafts are fabricated by layering several different types of materials such as metals, plastics, adhesives, and the like, with an innermost layer as a flat, wound monocoil spring. It is desireable to have endoscopic insertion shafts comprising a composite material to provide advantageous mechanical characteristics. However, current endoscope insertion shafts are often constructed using a composite method that employs only liquid resins as the binding and wear layer materials. Additionally, the substructures of the composite are most often a flat wound coil with an over sheath of coaxial braided wire. This results in three basic weaknesses: first, the liquid resins do not provide a durable lamination due to the partial cross-linking and tend to break down over time, losing their elasticity. Second, the liquid resins do not provide a durable wear layer due to the partial cross-linking and tend to break down or delaminate over time. Third, the coil substructure is inherently unstable and cannot provide bi-directional torsion stability or substantial hoop strength.

### Summary Of The Invention

The present invention comprises a tubular member as the innermost layer of an endoscope insertion shaft. The tubular member has greater flexibility and torsional strength, and an increased hoop strength providing crush resistance for the shaft. Additionally, the present invention provides for resistance to buckling, improved durability, and ease of manufacture of the endoscope shaft. The present invention also limits compression and provides for axial stability of the endoscope shaft, while allowing for adjustment in terms of stiffness of the shaft.

The tubular member of the present invention is preferably slotted to provide additional flexibility. The tubular member and the slots are of specific dimensions and material composition so as to achieve the desired degree of flexibility, resistance to kinking, torsional stability and axial stability. The slots are preferably provided as either radial slots, axial slots, or bias slots to relieve stresses and increase flexibility in or more desired planes of deflection. The slots are formed in the tube by cutting or the like. The cutting of slots may be radially indexed in any increment to achieve or limit the desired degree of planar bending.

The endoscope insertion shaft additionally comprises connections, interfaces, and end fittings such as joints and the like. The joints may be welded, bonded, or otherwise connected to the shaft. These fittings may be machined directly into the tubular member to reduce the number of total parts.

In one embodiment, the present invention comprises an endscope insertion shaft of a composite material, an adapter connected a proximal end of the shaft by a bonded joint, and a collar positioned at a distal end of the shaft by a welded joint. The composite material comprises an innermost tubular layer with slots, a polyester shrink layer, a braided layer which may be filled with urethane, and an outer sheath adhered with urethane.

### Brief Description Of The Drawings

Figure 1 is an illustration of an endoscope insertion shaft symmetric about an axis.

Figure 2 is a section of the distal end of the endoscope insertion shaft of Figure 1.

Figure 3 is an illustration of a tubular member of the endoscope insertion shaft of Figure 1 having a first pattern of apertures.

Figure 4 is an end view of the endoscope insertion shaft of Figure 3.

Figure 5 is an illustration of a tubular member of the endoscope insertion shaft of Figure 1 having a second pattern of apertures.

Figure 6 is an illustration of a tubular member of the endoscope insertion shaft of Figure 1 having a third pattern of apertures.

Figure 7 is an illustration of a tubular member of the endoscope insertion shaft of Figure 1 having a fourth pattern of apertures.

### Detailed Description Of The Invention

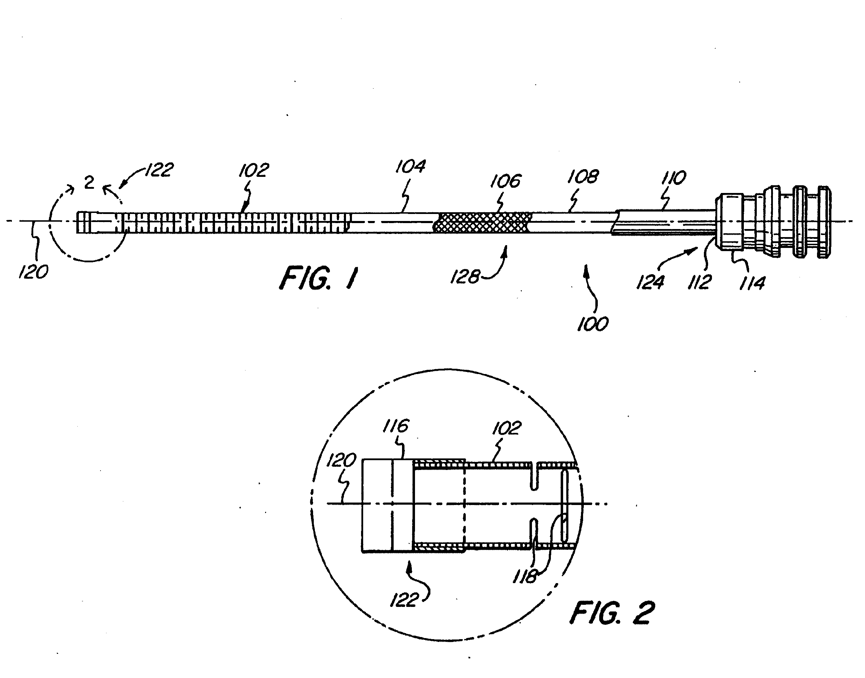

Figure 1 illustrates an endoscope insertion shaft 100 comprising a tubular member 102 having an axis 120. The endoscope insertion shaft 100 also comprises a distal end 122 (also shown in Fig. 2.) and a proximal end 124. The distal end 122 includes a collar 116 encompassing the tubular member 102. The proximal end 124 includes an adapter 114 for attaching the endoscope insertion shaft 100 to ancillary medical apparatus. The adapter 144 is connected to the tubular member 102 by a bonded joint 112.

The tubular member 102 includes at least one aperture or slot 118. The slots 118 may be of any size, configuration or orientation so as to provide desirable mechanical properties in the endoscope insertion shaft 100 such as torsional stability, hoop strength, beam strength, flexibility, elasticity and durometer (if applicable), etc. The spacing, arrangement, and location of the slots 118 in the endoscope insertion shaft 100 are such as to provide the desired degree of flexibility in the desired direction, such as in the longitudinal or axial directions. In one embodiment, the slots 118 extend over the entire length of the endoscope insertion shaft 100. In another embodiment, the slots 118 extend only over a portion of the length of the endoscope insertion shaft 100. The slots 118 may be arranged in different portions of the tubular member 102 of the endoscope insertion shaft 118.

The size, shape and orientation of the slots 118 in relation to the tubular member 102 may vary to render the desired flexibility of the endoscope insertion shaft 100. The slots 118 may be cut longitudinally, diagonally and axially into the tubular member 102. The slots 118 may be cut to different lengths and shapes, preferably in a straight line.

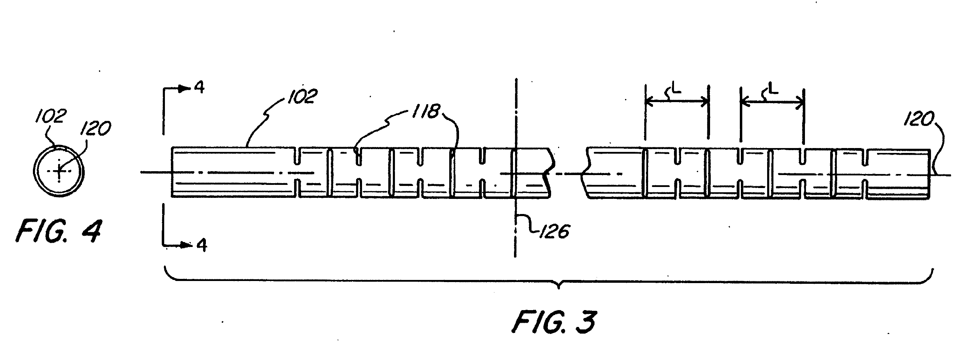

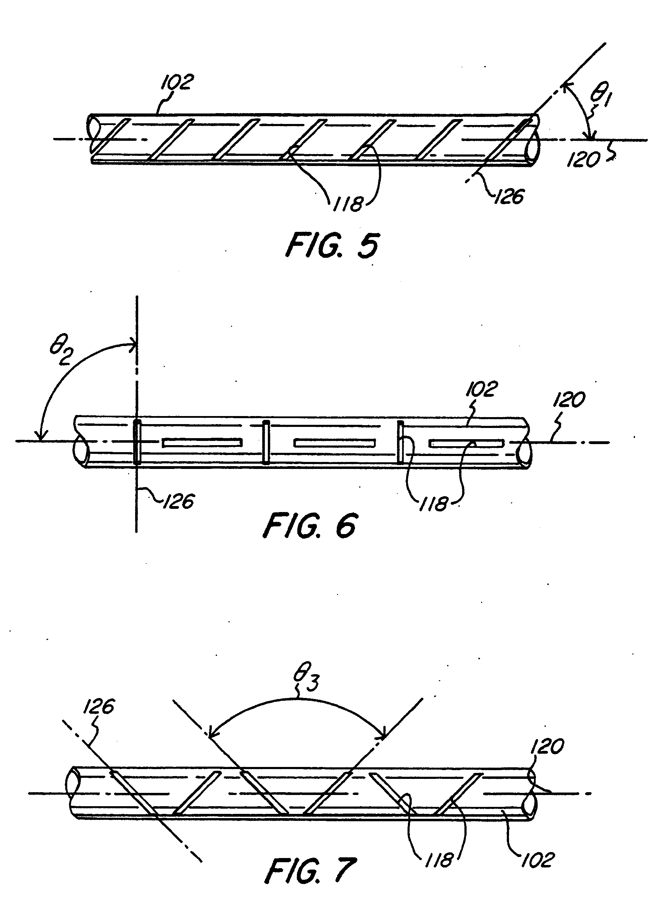

The slots 118 may be arranged in a regular and repeating fashion to form a specific pattern. In one embodiment, the slots 118 are cut into the tubular member 102 in radially matched pairs which subtend an arc of 180 degrees about the circumference of the tubular member 102. These pairs of slots 118 are arranged at a specific distance, L, from each other along the tubular member 102. As in Figures 2 and 3, along the tubular member, successive pairs of slots 118 may alternate circumferentially at a 90 degree angles from one another in the tubular member 102 to form the desired pattern. These pairs of slots 118 may be cut at the same angle, θ₁, with respect to the axis 120 as in Figures 5 and 6, or the pairs of slots 118 may be cut in different directions (θ₂, θ₃) and be alternated in the desired pattern as in Figures 6 and 7. For example, as in Figure 6, longitudinal cut pairs of slots 118 may alternate with axially cut pairs of slots 118 in the desired portion of the tubular member 102. It will also be understood that the slots 118 may also comprise any set of slots equally spaced about the circumference of the tubular member 102. For example, there may be three slots 118 spaced at 60 degrees from one another about the circumference of the tubular member 102 or four slots 118 spaced at 90 degrees from one another, etc.

The material of the tubular member 102 comprises for example stainless steel, austenitic stainless steel, martensitic stainless steel, Nitinol, nickel alloys, copper alloys or high modulus plastics such as polyimides.

The endoscope insertion shaft 100 also comprises a sleeve-like sheath 128 which includes an optional barrier layer 104, a braided layer 106, a laminating layer 108 and a wear layer 110 encasing or jacketing the tubular member 102. The barrier layer 104 comprises a polyester shrink wrap. The braided layer 106 in turn jackets the barrier layer 104 and the tubular member 102. The material of the braided layer 106 comprises for example austenitic stainless steel, carbon fiber, aramides or aramide fibers such as KEVLAR® fiber. The laminating layer 108 jackets the braided layer 106, the barrier layer 104 and the tubular member 102. The material of the laminating layer 108 comprises a urethane such as a polyurethane epoxy or an extruded polyetherurethane, as well as a polyester resin or a polyimide. The wear layer 110 in turn jackets the laminating layer 108, the braided layer 106, the barrier layer 104 and the tubular member 102. The wear layer 110 comprises an extruded polyetherurethane. Thus, the tubular member 102, the barrier layer 104, the braided layer 106, the laminating layer 108 and the wear layer 110 form a composite material.

A first method of manufacturing the composite endoscope insertion shaft 100 of the present invention comprises a cold lamination method. This method uses a technique of layering tubular structures with desirable characteristics (i.e. torsional stability, hoop strength, beam strength, flexibility, durometer, etc) and embedding these layers with a liquid resin to form a bond between layers. The resin cures via a chemical reaction to form a partially cross-linked polymer that can be tailored to the desired flexibility characteristic. As a final layer, a thermoplastic tube is applied in order to achieve a durable outer wear surface. The result of this layering is an endoscope insertion shaft that exhibits the additive characteristics of the individual layers in a single structure.

A second method of manufacturing the composite endoscope insertion shaft 100 of the present invention comprises a hot fusion method (thermoplastic, fully cross-linked). This method uses a technique of layering tubular structures with desirable mechanical characteristics (i.e. torsion, hoop strength, beam strength, flexibility, durometer, etc) and embedding these layers by re-flowing a thermoset plastic, under high heat conditions to form a bond between layers. The plastic cools as a fully cross-linked polymer that can be tailored to the desired flexibility characteristic. The result of this layering is a composite endoscope insertion shaft that exhibits the additive characteristics of the individual layers in a single composite structure.

The advantage of the hot fusion method over the cold lamination method is four fold: first, fully cross linked polymers are much more durable, second, the process is environmentally and people friendly, third, the process is less costly due to the elimination of the epoxy resins and subsequent operations to cure them properly and fourth, the process allows more layers of polymers to be fused together.

Using the methods above, endoscope insertion shafts are improved by beginning the composite lay-up with an inherently stabile substructure, and then building upon it with cross-linked polymers that provide long-term durability. Mechanical characteristics that are achieved and maintained are: torsional strength, beam strength, elasticity (in bending), longitudinal stability, compression strength, column stiffness, resistance to buckling, hoop strength (crush resistance), lightweight, lubricity, biocompatibility, color, graduation or change of properties along the length of the shaft. No single material can provide these properties or the ability to change the composition at will.

Thus based upon the foregoing description an endoscope insertion shaft is disclosed comprising a tubular member having an axis and including at least one aperture for imparting desirable mechanical characteristics to the shaft; and a composite, laminated or fused material comprising at least one layer encasing the tubular member.

It should be understood that any reference to first, second, front, rear, etc. or any other phrase indicating the relative position of one element or device with respect to another is for the purposes of explanation of the invention and, unless other wise noted, is not to be construed as limiting the invention. Furthermore, while preferred embodiments have been shown and described, various modifications may be made thereto without departing from the true spirit and scope of the invention. Accordingly, it is to be understood that the present invention has been described by way of illustration and not limitation.

## Claims

1. An endoscope insertion shaft comprising:
a tubular member (102) having an axis (120) and including at least one aperture (118) for increasing the flexibility thereof; and
a sheath (128) comprising at least one layer (104, 106, 108, 110) jacketing the tubular member (102) and comprising polyesters, polyester resins, austenitic stainless steel, carbon fibers, aramides, aramide fibers, polyurethane epoxies, extruded polyetherurethane or polyimides.

2. The endoscope insertion shaft of claim 1, wherein the at least one aperture (118) comprises a pattern of apertures (118).

3. The endoscope insertion shaft of claim 2, wherein the pattern of apertures (118) comprises a first set of apertures (118) positioned along a line parallel to the axis of the tubular member (102).

4. The endoscope insertion shaft of claim 3, wherein the first set of apertures (118) comprises at least one elongated aperture (118) having an axis oriented at an angle to the axis (120) of the tubular member (102).

5. The endoscope insertion shaft of claim 4, wherein the angle is in the range from zero to ninety degrees.

6. The endoscope insertion shaft of anyone of claims 2 through 5, wherein the pattern of apertures (118) comprises a pair of apertures (118).

7. The endoscope insertion shaft of anyone of claims 2 through 6, wherein the apertures (118) are circumferentially positioned on the tubular member (102).

8. The endoscope insertion shaft of anyone of claims 1 through 7, wherein the at least one layer comprises:
a braided layer (106) jacketing the tubular member (102); and
a laminating layer (108) jacketing the tubular member (102).

9. The endoscope insertion shaft of claim 8, further comprising a barrier layer (104) jacketing the tubular member (102).

10. The endoscope insertion shaft of claim 8 or 9, wherein the laminating layer (108) jackets the braided layer (106).

11. The endoscope insertion shaft of anyone of claims 8 through 10 wherein a wear layer (110) jackets the laminating layer (108).

12. The endoscope insertion shaft of anyone of claims 1 through 11, wherein the sheath (128) comprises a composite material.

13. An endoscope insertion shaft, in particular according to anyone of claims 1 through 12, comprising:
a composite body having an axis (120) and at least one aperture (118) for increasing the flexibility thereof and comprising austenitic stainless steel, martensitic stainless steel, Nitinol, nickel alloys, copper alloys, high modulus plastics, polyesters, polyester resins, carbon fibers, aramides, aramide fibers, polyurethane epoxies, extruded polyetherurethanes or polyimides.

14. An endoscope, comprising an endoscope insertion shaft (100) according to anyone of claims 1 through 13.

15. A method of manufacturing an endoscope insertion shaft the method comprising:
layering a plurality tubular structures, each tubular structure having prescribed mechanical properties;
under a prescribed temperature and pressure, forming a bond between the tubular structures; and
cooling the tubular structures to form a cross linked polymer.

16. The method of claim 15, wherein forming a bond comprises reflowing a thermoset plastic.
